Europäisches Patentamt

European Patent Office

Office européen des brevets

(11) Publication number: **0 241 242 B1**

## EUROPEAN PATENT SPECIFICATION

(45) Date of publication of patent specification: **11.03.92**    (51) Int. Cl.5: **A61K 37/66**

(21) Application number: **87302945.8**

(22) Date of filing: **03.04.87**

(54) The use of interferon-beta and interleukin-2 for combination therapy and compositions therefor.

(30) Priority: **03.04.86 US 847509**
     **16.01.87 US 4108**

(43) Date of publication of application:
     **14.10.87 Bulletin  87/42**

(45) Publication of the grant of the patent:
     **11.03.92 Bulletin  92/11**

(84) Designated Contracting States:
     **AT BE CH DE ES FR GB GR IT LI LU NL SE**

(56) References cited:
     **EP-A- 170 843**
     **EP-A- 0 149 551**
     **US-A- 4 530 787**

**CHEMICAL ABSTRACTS, vol. 105, no. 23, 8th December 1986, page 468, abstract no. 207396r, Columbus, Ohio, US; M. IIGO et al.: "Synergistic inhibition of the growth of adenocarcinoma 755 by the combination of interleukin-2 and interferon-beta", & PROC. JPN. ACAD., SER. B 1986, 62(7), 275-8**

**JOURNAL OF IMMUNOLOGY, Vol. 126, n. 6, June 1981, pp. 2321-2327**

**Cancer Research 41, pp. 4420-4425, 1981**

**Journal Experimental Medicine, 155, pp. 1823-1841, 1982**

**Journal of Immunology, Vol. 135, N 2, August 1985, pp. 1145-1152**

(73) Proprietor: **CETUS CORPORATION**
     **1400 Fifty-Third Street**
     **Emeryville California 94608(US)**

(72) Inventor: **Rudolph, Alfred**
     **258 Claudia Court**
     **Moraga California 94556(US)**

(74) Representative: **Bizley, Richard Edward et al**
     **HEPWORTH LAWRENCE BRYER & BIZLEY**
     **2nd Floor Gate House South West Gate**
     **Harlow, Essex CM20 1JN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a combination of interferon-$\beta$ and interleukin-2 and the use of this combination as an anti-tumor therapeutic or prophylactic agent.

Interleukin-2, a lymphokine which is produced by normal peripheral blood lymphocytes and induces proliferation of antigen or mitogen stimulated T cells after exposure to plant lectins, antigens, or other stimuli, was first described by Morgan, D. A., et al., Science (1976), 193:1OO7-1OO8. Then called T cell growth factor because of its ability to induce proliferation of stimulated T lymphocytes, it is now recognized that in addition to its growth factor properties it modulates a variety of functions of immune system cells in vitro and in vivo and has been renamed interleukin-2 (IL-2).

IL-2 was initially made by cultivating human peripheral blood lymphocytes (PBL) or other IL-2-producing cell lines. See, for example, U.S. Patent No. 4,4O1,756. Recombinant DNA technology has provided an alternative to PBLs and cell lines for producing IL-2. Taniguchi, T. et al., Nature (1983), 3O2:3O5-31O and Devos, R., Nucleic Acids Research (1983), 11:43O7-4323 have reported cloning the human IL-2 gene and expressing it in microorganisms.

U.S. Patent No. 4,518,584 describes and claims muteins of IL-2 in which the cysteine normally occurring at position 125 of the wild-type or native molecule has been replaced with a neutral amino acid, such as serine or alanine. U.S. Patent Nos. 4,53O,787 and 4,569,79O disclose and claim methods for purifying recombinant native IL-2 and muteins thereof, as well as the purified form of IL-2.

PCT WO85/O4328 discloses an IL-2 composition suitable for reconstituting in a pharmaceutically acceptable aqueous vehicle composed of oxidized microbially produced recombinant IL-2. The IL-2 is noted as useful in combination with cytotoxic chemotherapy or irradiation or surgery in the treatment of malignant or pre-malignant diseases in a direct therapeutic or adjuvant setting or in combination with other immune-modulating drugs, lymphokines (e.g., IL-1, IL-3, CSF-1 and IFNs) naturally occurring, or inducible anti-cellular toxins in treating malignant diseases.

Various therapeutic applications of human IL-2 have been investigated and reported by S. Rosenberg and colleagues (see Mule et al., Science (1984), 225:1487 and S. Rosenberg et al., New England Journal of Medicine (1985), 313:1485-1492, for example).

Interferons (IFN) constitute a group of naturally occurring proteins which are known to exhibit anti-viral, anti-tumor and immunoregulatory behavior. Two types of IFN have been identified based on differences in their observed biological properties and molecular structures: Type I and Type II. Beta-interferon (IFN-$\beta$) is a Type I IFN which can be induced in fibroblasts by viral challenge and contains about 165 amino acids. IFN-$\alpha$ is also a Type I IFN inducible in leukocytes, and IFN-$\gamma$ is a Type II IFN which is induced in lymphocytes in response to specific mitogenic stimuli and contains 146 amino acids.

Human IFN-$\beta$ may be produced by recombinant DNA technology, as described, for example, in EP 28,O33 published June 6, 1981 to Sugano, et al. and U.K. 2,O63,882 published June 1O, 1981 to Revel, et al. Additionally, the IFN-$\beta$ may be a mutein in which amino acids not essential to biological activity are deleted or replaced with other amino acids to increase stability, as described by U.S. Patent No. 4,588,585.

After Paucker et al., Virology, 17:324-334 (1962) showed that IFN suppressed the growth rate of mouse L cells, many investigators have studied treatment of mouse L cells with IFN and inhibition of tumor cell proliferation by IFN. See, e.g., Borden, E. C., Ann. Intern. Med., 91:472-479 (1979).

Combination chemotherapy using two or more anti-cancer drugs to treat malignant tumors in humans is currently in use in research and in the clinic. The anti-cancer drugs may be antimetabolites, alkylating agents, antibiotics, general poisons, etc. Combinations of drugs are administered in an attempt to obtain a synergistic cytotoxic effect on most cancers, e.g., carcinomas, melanomas, lymphomas and sarcomas, and to reduce or eliminate emergence of drug-resistant cells and to reduce side effects to each drug.

It is known that Type I and Type II interferons may be combined to produce a synergistic biological effect. See, for example, Fleishmann, W. R., Cancer Res. (1982), 42:869-875 and DeClercq, E., et al., Cancer Letters (1982), 15:223-228 (mouse IFNs), and European Patent Publication 1O7,498 published May 2, 1984 (human IFN-$\gamma$ and IFN-$\alpha$ or -$\beta$). U.S. Patent 4,518,584 to Mark et al. (Cetus Corporation) discloses the combination of IL-2 muteins with gamma-interferon, B cell growth factor, and IL-1. In addition, it has been disclosed that IL-2 may be used with IFN-$\gamma$ to treat tumor-bearing hosts with synergistic results (European Patent Publication 149,551 published July 24, 1985 (Genentech) and German Patent Publication 3411184 published October 31, 1985 (Deut Roten Kreuzes)) or with augmentation of natural killer activity (Svedersky et al., J. Immunol. (1984), 133:714-718 and Shalaby et al., J. Interferon Res. (1985), 5:571-581). Lopez-Botet et al., Eur. J. Immunol. (1984), 14:1137-1141 reported, however, that IL-2 and IFN-$\gamma$ are not sufficient in combination to induce natural killer-like activity in human T cell clones. It is also known from Dempsey et al., J. Immun. (1982), 129:25O4-251O that the combination of IFN-$\alpha$ and IL-2 is more effective

2

EP 0 241 242 B1

than IFN-α or IL-2 alone in causing natural killer cell activation. Also, Dr. Talmadge of the Preclinical Screening Lab., BRMP has reported in 1986 the augmented effect of using TNF and IFN-γ to treat metastatic disease in mice. EP 131,789, published January 23, 1985 (Sloan-Kettering Institute for Cancer Research) and EP 168,214, published January 15, 1986 (Genentech), disclose the synergistic effect of TNF and IFN-γ to treat various tumors in mice.

Moreover, U.S. Statutory Invention Registration No. H22, published February 4, 1986 to Creasey et al. discloses a composition exhibiting a synergistic cytotoxic effect in combination therapy of certain breast cancer and myeloma cell lines using synergistically effective amounts of 5-fluorouracil and human recombinant beta-interferon.

Brooks et al, J. of Immunology, Vol. 135, N°2, Aug. 1985, pp. 1145-1152 teach that the combination of IFN-β and IL-2 activates NK cells in vitro, whereas this is not the case for IL-2 and IFN-γ. LOTZE et al, Cancer Research 41, pp. 4420-4425 (1981) and GRIMM et al, Journal Experiment Medicine, 155, 1823-1841 (1982) have shown that there is an uncertain correlation between NK-cells activities and actual antitumor activity.

The effect of a combination of IL-2 and IFN-β on tumor-bearing animals has not been studied, to the applicant's knowledge.

Accordingly, the present invention provides a composition suitable for administration to human patients for therapeutic or prophylactic treatment of cancer comprising a mixture of IFN-β and IL-2 in a carrier at a concentration of about 0.1mg/ml to 100mg/ml, wherein the IFN-β and IL-2 are from mammalian species.

In another aspect, the invention includes human recombinant IFN-β and IL-2 at the above concentration for use together in a method for therapeutic or prophylactic treatment of cancer in human patients.

The invention further provides the use of mammalian IFN-β and IL-2 at the above concentrations in the manufacture of a medicament for the prophylaxis or treatment of cancer, wherein the medicament comprises a mixture of the IFN-β and IL-2.

In yet another aspect, the invention includes a method of making a therapeutic or prophylactic composition suitable for administration to human patients for therapeutic or prophylactic treatment of cancer comprising formulating together, whether by mixing or providing separate doses or otherwise, mammalian IFN-β and IL-2.

The invention thus enables a method for therapeutic or prophylactic treatment of cancer in human patients comprising administering an effective amount of IFN-β and IL-2 to the patient, wherein the IFN-β and IL-2 are from mammalian species.

Preferably the IFN-β and IL-2 are recombinant, microbially produced human proteins.

The combination of IFN-β and IL-2 provides effective treatment of various forms of cancer such as melanomas, leukemia, renal cell cancer and lung cancer.

As used herein, the term "therapeutic" treatment refers to administration to the patient of the IFN-β and IL-2 after the patient has contracted cancer, as determined by any means. The treatment is not considered therapeutic if after treatment a tumor appears or an existing tumor burden is not decreased or eliminated. The term "prophylatic" treatment refers to administration to the patient of the IFN-β and IL-2 after the patient has been treated for cancer, to prevent reoccurrence of the cancer.

As used herein, the term "cancer" refers to any neoplastic disorder, including such cellular disorders as, for example, renal cell cancer, Kaposi's sarcoma, chronic leukemia, breast cancer, sarcoma, ovarian carcinoma, rectal cancer, throat cancer, melanoma, colon cancer, bladder cancer, matocytoma, lung cancer and gastrointestinal or stomach cancer. Preferably, the cancer is colon cancer, melanoma, renal cell cancer, lung cancer, adenocarcinoma, or breast cancer.

As used herein, the term "recombinant" refers to IL-2 produced by recombinant DNA techniques wherein generally the gene coding for the IL-2 is cloned by known recombinant DNA technology. For example, by using the human IL-2 cDNA as a template, the gene showing complementarity to the human IL-2 cDNA is inserted into a suitable DNA vector such as a bacterial plasmid, preferably E. coli plasmid, to obtain a recombinant plasmid, and the plasmid is used to transform a suitable host. The gene is expressed in the host to produce the recombinant protein. Examples of suitable recombinant plasmids for this purpose include pBR322, pCR1, pMB9 and pSC1. The transformed host may be eucaryotic or procaryotic, preferably a procaryotic host.

As used herein, the term "pharmaceutically acceptable" refers to a carrier medium which does not interfere with the effectiveness of the biological activity of the active ingredients and which is not toxic to the hosts to which it is administered.

The IL-2 and IFN-β may be combined in vitro before administration or separately administered to the patient, in either order or simultaneously. An example is the protocol set forth in Example 1 hereof, wherein the IL-2 and IFN-β are administered within minutes of each other.

3

The administration(s) may take place by any suitable technique, including parenteral administration. Examples of parenteral administration include intravenous, intraarterial, intramuscular, and intraperitoneal, with intravenous, intramuscular and subcutaneous administration being preferred, and with intravenous administration of IFN-$\beta$ and intravenous or subcutaneous administration of IL-2 being most preferred.

The dose and dosage regimen will depend mainly on whether the IL-2 and IFN-$\beta$ are being administered separately, i.e., concurrently, such as same day, or sequentially, such as alternate days or weeks) or as a mixture, the type of cancer, the patient, and the patient's history. The amount must be effective to achieve some tumor reduction or augmentation of LAK activity. The doses may be single doses or multiple doses. If multiple doses are employed, as preferred, the frequency of administration will depend, for example, on the type of cancer, dosage amounts, etc. For some types of cancers, daily administration may be effective, whereas for others, administration every other day or every third day may be effective, but daily administration ineffective. The practitioner will be able to ascertain upon routine experimentation which route of administration and frequency of administration are most effective in any particular case.

The dosage amount which appears to be most effective herein is one which results in regression in size of the tumor or complete disappearance or non-reappearance of the tumor, and is not toxic or is acceptably toxic to the patient, as defined by the protocol in Example 1. Generally, such conditions as fever, chills and general malaise are considered acceptable. This optimum dose level will depend on many factors, for example, on the type of cancer, route, schedule and sequence of administration, existing tumor burden, the type of IL-2 and IFN-$\beta$, and the definition of toxicity. Toxicity may be defined by the extent and type of side effects, with fever, chills and general malaise considered acceptable toxicity for the study herein.

If there is acceptable toxicity and if the route of administration is separate administration of IFN-$\beta$ and IL-2 three times a week, the dosage level for each administration of recombinant, microbially produced IL-2 and IFN-$\beta$ is preferably about $10^4$ to $10^7$ units IL-2 per square meter of body surface area ($M^2$) (more preferably about $10^6$ to $10^7$ units/$M^2$) and from about 1 to $400 \times 10^6$ units IFN-$\beta$ per $M^2$ (more preferably $10^6$-$10^7$ units/$M^2$).

For parenteral administration the IL-2 and IFN-$\beta$ will generally be formulated in a unit dosage injectable form (solution, suspension, emulsion), preferably in a pharmaceutically acceptable carrier medium which is inherently non-toxic and non-therapeutic. Examples of such vehicles include saline, Ringer's solution, dextrose solution, mannitol, and normal serum albumin. Non-aqueous vehicles such as fixed oils and ethyl oleate may also be used. The carrier medium may contain minor amounts of additives such as substances which enhance isotonicity and chemical stability, e.g., buffers and preservatives. The IL-2 and IFN-$\beta$ will preferably be formulated in such carriers at a concentration of about 0.2 to 1 mg/ml of each.

Alternatively, the IL-2 and IFN-$\beta$ may be made into a sterile, stable lyophilized formulation in which the purified IL-2 and IFN-$\beta$ are admixed with a water-soluble carrier such as mannitol, which provides bulk, and a sufficient amount of sodium dodecyl sulfate to ensure the solubility of the recombinant IL-2 and IFN-$\beta$ in water. The formulation is suitable for reconstitution in aqueous injections for parenteral administration and it is stable and well-tolerated in human patients. The formulation method is more completely described in PCT WO85/04328.

In yet another alternative, the mixture of IFN-$\beta$ and IL-2 may be administered in an adoptive immunotherapy method, together with isolated, lymphokine-activated lymphocytes in a pharmaceutically acceptable carrier, where the lymphocytes are reactive to tumor when administered with the IFN-B and IL-2 to humans suffering from the tumor. This method is described more fully in S. Rosenberg et al., New England Journal of Medicine (1985), 313:1485-1492.

As mentioned above, the IL-2 and IFN-$\beta$ herein may be any IL-2 and IFN-$\beta$ prepared from tissue cultures or by recombinant techniques, and from any mammalian source such as, e.g., mouse, rat, rabbit, primate, pig, and human. Preferably the IFN-$\beta$ and IL-2 are from a human source. More preferably the IFN-$\beta$ and IL-2 are recombinant.

The recombinant IL-2 may be obtained as described by Taniguchi et al., Nature, 302:305-310 (1983) and Devos, Nucleic Acids Research, 11:4307-4323 (1983) by cloning the native human IL-2 gene and expressing it in transformed microorganisms. It may also be an IL-2 mutein as described in U.S. Patent No. 4,518,584, in which the cysteine normally occurring at position 125 of the wild-type or native molecule has been deleted or replaced by a neutral amino acid such as serine or alanine, or an IL-2 mutein in which the methionine normally occurring at position 104 or the wild-type or native molecule has been replaced by a neutral amino acid such as alanine.

Preferably, the IL-2 is an unglycosylated protein which is produced by a microorganism which has been transformed with the human cDNA sequence or a modified human cDNA sequence of IL-2 which encodes a protein with an amino acid sequence at least substantially identical to the amino acid sequence of native human IL-2, including the disulfide bond of the cysteines at positions 58 and 105, and has biological

activity which is common to native human IL-2. Substantial identity of amino acid sequences means the sequences are identical or differ by one or more amino acid alterations (deletions, additions, substitutions) which do not cause an adverse functional dissimilarity between the synthetic protein and native human IL-2. Examples of IL-2 proteins with such properties include those described by Taniguchi et al., Nature (1983), 302:305-310; Devos, Nucleic Acids Research (1983), 11:4307-4323; and by European Patent Publication Nos. 91,539 and 88,195; and in U.S. Patent 4,518,584, supra. Most preferably, the IL-2 is the des-ala$_1$-IL-2$_{ser125}$ mutein in which the initial terminal alanine is deleted and the cysteine at position 125 is replaced by a serine residue. The IL-2 employed may have at least one or more of the first five N-terminal amino acids of the native IL-2 deleted.

The IL-2 may be purified to clinical purity by the method described in U.S. Patent No. 4,569,790, issued February 11, 1986.

In an alternative formulation, the IL-2 may be solubilized, not by a detergent, but by reacting the IL-2 with an activated polymer selected from polyethylene glycol or polyethylated polyol. The polymer is activated by conjugation with a coupling agent having terminal groups reactive with both the free amino or thiol groups of the IL-2 and the hydroxyl group of the homopolymer. Examples of such coupling agents include hydroxynitrobenzene sulfonic ester, cyanuric acid chloride, and N-hydroxysuccinimide. This modification eliminates the necessity for adding detergents to solubilize the IL-2 at physiological pH. The IL-2 is then formulated directly with the water-soluble carrier and buffer as described above, and the formulation may be lyophilized and the lyophilized mixture reconstituted as described above.

The IFN-$\beta$ herein may be produced naturally by cells exposed to interferon inducers such as viruses or double-stranded polyribonucleotides, as taught by Metz, Adv. Drug Res., 10:101-156 (1975). IFN-$\beta$ may also be made by recombinant means such as the method disclosed by EP 28,033 published June 6, 1981. Muteins of IFN-$\beta$ may also be prepared as described by U.S. Patent No. 4,588,585. In particular, a preferred IFN-$\beta$ mutein is IFN-$\beta_{ser17}$, which is not glycosylated, lacks the N-terminal methionine, and has the cysteine residue at position 17 of native IFN-$\beta$ replaced by serine using site-specific mutagenesis. The IFN-$\beta$ may be produced and purified by the method described in U.S. Patent No. 4,462,940.

The various aspects of the invention are further described by the following examples, which are not intended to limit the invention in any manner. In these examples all parts for solids are by weight and all percentages for liquids and gases are by volume, unless otherwise noted, and all temperatures are given in degrees Celsius.

EXAMPLE 1

A. General Treatment Plan

1. Patients

The total number of human patients studied was 97. Each patient had a specific type of cancer and were male and female above 18 years of age. In addition, the patients fulfilled all of the following criteria to be eligible for study admission:

1. A primary diagnosis of histologically proven solid tumor malignancy refractory to standard anti-cancer therapy or for which there is no generally accepted therapy;

2. Patients must be ambulatory with a Karnofsky performance status of >70 or more (ECOG PS 0 or 1);

3. Creatinine less than 2.0 mg/100 ml;

4. Bilirubin less than 1.5 mg/100 ml; prothrombin time less than 1.3 × control;

5. Granulocytes over 1000/mm$^3$; platelets over 50,000/mm$^3$;

6. A minimum life expectancy of three months; and

7. The ability to give written informed consent which must be obtained prior to treatment.

Any of the following patients were excluded from the study:

1. Patients with clinically significant cardiac disease, i.e., New York Heart Association Class III or IV.

2. Patients with serious active infections requiring antibiotic therapy.

3. Pregnant or lactating women of childbearing potential unless using effective contraception.

4. Subjects who have participated in another experimental clinical trial within three weeks of entry into the present study.

5. Patients with CNS metastases, CNS infection (including retinal disease), vasculitis, a known seizure disorder, or a T-cell malignancy.

6. Preadmission medication or other treatment exclusions:

a. Chemotherapy, hormonal therapy, immunotherapy or radiation therapy will have been withheld for a minimum of 3 weeks prior to study entry (6 weeks for nitrosoureas or mitomycin C).

b. Patients requiring ongoing therapy with corticosteroids or nonsteroidal anti-inflammatory drugs will not be eligible.

c. The patient should be considered fully recovered from any prior surgical treatment.

7. Patients with organ allografts.

### 2. IL-2

The recombinant IL-2 employed in this example was des-ala$_1$-IL-2$_{ser125}$. The amino acid sequence of this IL-2 differs from the amino acid sequence of native human IL-2 in that it lacks the initial alanine of the native molecule, and the cysteine at position 125 has been changed to serine. Samples of E. coli that produce this IL-2 have been deposited by Cetus Corporation in the American Type Culture Collection, 1201 Parklawn Drive, Rockville, Md, USA on September 26, 1983 under accession number 39,452 and on March 6, 1984 under accession number 39,626 under the provisions of the Budapest Treaty.

The IL-2 was processed and purified as described in the text and Figure 1 of PCT WO85/04328, except that the oxidation was carried out using copper chloride, as described in U.S. Patent No. 4,572,798 rather than o-iodosobenzoate. When the IL-2 was recovered from the chromatography step(s) it was lyophilized and resuspended in a neutral aqueous buffer containing the reducing agent (DTT) to keep the IL-2 in a reduced state and a solubilizing agent to keep it in solution. The purity of the recombinant IL-2 after the chromatography step(s) was at least about 95% and the IL-2 contained less than about 0.02 ng/ml endotoxin as determined by the Limulus amebocyte assay.

The purified IL-2 was formulated at a concentration of 0.3 mg/ml with 50 mg/ml mannitol.

### 3. IFN-$\beta$

The recombinant IFN-$\beta$ employed in this example was IFN-$\beta_{ser17}$. The amino acid sequence of this IFN-$\beta$ differs from that of native human IFN-$\beta$ in that the cysteine at position 17 has been changed to serine. Samples of E. coli that produce this IFN-$\beta$ have been deposited by Cetus Corporation in the American Type Culture Collection on November 18, 1983 under accession number 39,517.

The IFN-$\beta$ was processed and purified as described in Example I of U.S. Statutory Invention Registration No. H22 published February 4, 1986. The purified IFN-$\beta$ was formulated at a concentration of 0.25 mg/ml with 12.5 mg/ml of normal serum albumin and 12.5 mg/ml of dextrose.

### 4. Treatment Plan

Patients were treated simultaneously with 3 times weekly IL-2 given intravenously or subcutaneously and 3 times weekly IFN-$\beta$ given as a bolus intravenous injection on Monday, Wednesday and Friday of each week. Treatment was continued for a minimum of one month in the absence of progressive disease or unacceptable toxicity. Four patients were entered at each dose level before additional patients were entered at a higher dose on the following schema:

| Level | IL-2 (units/M$^2$) | IFN-$\beta$ (units/M$^2$) |
|-------|--------------------|---------------------------|
| I | $1 \times 10^4$ | $2 \times 10^6$ |
| II | $1 \times 10^5$ | $2 \times 10^6$ |
| III | $1 \times 10^6$ | $2 \times 10^6$ |
| IV | $5 \times 10^6$ | $2 \times 10^6$ |
| V | $5 \times 10^6$ | $6 \times 10^6$ |
| VI | $5 \times 10^6$ | $10 \times 10^6$ |
| VII | $5 \times 10^6$ | $20 \times 10^6$ |

Because neutralizing antibodies to the IL-2 developed using subcutaneous IL-2 administration, IL-2 was administered intravenously in later studies.

### 5. Concomitant Therapy

The following were excluded while the patients were on study:

1. Aspirin

2. Corticosteroids

3. Cimetidine, ranitidine

4. Barbiturates

5. Immunotherapy, hormonal therapy, chemotherapy or radiation therapy directed at treatment of the primary malignancy

6. Other investigational drugs

6. Response Criteria

Response was determined by measuring the longest diameter of all measurable lesions, measuring the length of the perpendicular line through this diameter, and multiplying these two lengths.

1. Complete Response

Disappearance of all clinical and laboratory signs of active disease for a minimum of four weeks.

2. Partial Response

A minimum of 5O% reduction in the multiplication product of the perpendicular diameters of all measurable lesions.

3. Stable Disease

From above 5O% reduction in the multiplication product to less than a 25% increase in the multiplication product of the perpendicular diameter of all measurable lesions, or no change in measurable lesions qualifying as a partial response or disease progression.

4. Progressive Disease

A 25% or greater increase in the multiplication product of the perpendicular diameters of all measurable lesions, or development of new lesions.

B. Results in Phase I Studies

Two separate clinical studies were conducted, one by Robert L. Krigel at Fox Chase Cancer Center, Philadelphia, PA with 47 patients, and one by Bernard Poiesz at SUNY Upstate Medical Center, Syracuse NY with 5O patients. The tumor types studied included colon, melanoma, renal cell, lung, adenocarcinoma and breast.

The NK and LAK activity of the patients were studied. For NK activity measurement, fresh mononuclear cells of each patient were plated for four hours on a Daudi cell line and put on chromium-labeled targets and the radioactivity of each was counted. The specific cell lysis was measured. For LAK activity measurement, the same procedure was followed except that the cells were incubated with des-ala$_1$-IL-2$_{ser125}$ for 24 hours before being placed on the Daudi cell line.

For the first study, a correlation could be made between LAK activity (measured by at least 25% cell lysis) and some activity against the tumor. One patient with melanoma indicated a partial response, and that patient had increased LAK activity. In addition, most patients demonstrating LAK activity also had some evidence of anti-tumor activity clinically (e.g., stable disease). It was found that the maximum tolerated dose of IL-2 was 5 x 10$^6$ units/M$^2$ due to volume limitations, and the maximum tolerated dose of IFN-$\beta$ was 10 x 10$^6$ units/M$^2$.

Regarding pharmacokinetics, naive patients showed IL-2 blood levels at 1-2 hours which persisted through six hours.

As to significant adverse effects, the following results were otained.

TABLE I

| Adverse Effect | Number of Patients (# Drug Related) | | | |
|---|---|---|---|---|
| | Study 1 (of 47) | | Study 2 (of 38) | |
| | Mild or Moderate | Severe or Life Threatening | Mild or Moderate | Severe or Life Threatening |
| Early deaths | -- | 6 (1) | 0 | 6 (0) |
| Hypotension | 6 (6) | 0 | 6 (5) | 1 (1) |
| Fever, chills | 40 (40) | 25 (25) | 28 (28) | 12 (12) |
| Rash | 8 (6) | 0 | 2 (2) | 1 (1) |
| Weight loss (>5%) | 4 (4) | 10 (10) | 0 | 0 |

In summary, the preliminary Phase I human clinical studies indicate that the combined treatment of IFN-$\beta$ and IL-2 has anti-tumor activity.

**Claims**

1. A medication suitable for administration to human patients for therapeutic or prophylactic treatment of cancer comprising IFN-beta and IL-2 each in a carrier at a concentration of about 0.1 mg/ml to 100 mg/ml, wherein the IFN-beta and IL-2 are from mammalian species.

2. A medication according to Claim 1, wherein the IFN-beta and IL-2 are human recombinant IFN-beta and IL-2.

3. A medication according to Claim 1 or Claim 2 wherein the IFN-beta is IFN-beta$_{ser17}$ and the IL-2 is des-ala$_1$-IL2$_{ser125}$.

4. A medication according to any one of Claims 1 to 3, wherein the cancer is colon cancer, melanoma, renal cell cancer, lung cancer, adenocarcinoma or breast cancer.

5. The use of mammalian IFN-beta and IL-2 in concentrations as defined in Claim 1 in the manufacture of a medicament for the prophylaxis or treatment of cancer.

6. The use according to Claim 5, wherein the medication comprises a mixture of the IFN-beta and IL-2.

7. The use according to Claim 5, wherein the medicament is a combined preparation for separate administration of IFN-beta and IL-2.

8. Products containing IFN-beta and IL-2 as a combined preparation for simultaneous, separate or sequential use in any therapeutic or prophylactic treatment of human cancer which employs said IFN-beta and IL-2 in concentrations as defined in Claim 1.

**Revendications**

1. Médicament approprié à l'administration à des patients humains pour le traitement thérapeutique ou prophylactique d'un cancer, comprenant de l'IFN-$\beta$ et de l'IL-2, chacun dans un véhicule, à une concentration d'environ 0,1 mg/ml à 100 mg/ml, dans lequel l'IFN-$\beta$ et l'IL-2 proviennent de mammifères.

2. Médicament selon la revendication 1, dans lequel l'IFN-$\beta$ et l'IL-2 sont de l'IFN-$\beta$ et de l'IL-2 humains recombinants.

3. Médicament selon la revendication 1 ou la revendication 2, dans lequel l'IFN-$\beta$ est l'IFN-$\beta_{ser17}$ et l'IL-2 est la dés-ala$_1$-IL-2$_{ser125}$.

4. Médicament selon l'une quelconque des revendications 1 à 3, dans lequel le cancer est un cancer du côlon, un mélanome, un cancer des cellules rénales, un cancer du poumon, un adénocarcinome ou un cancer du sein.

**5.** Utilisation d'IFN-$\beta$ et d'IL-2 de mammifères, aux concentrations définies dans la revendication 1, dans la préparation d'un médicament pour la prophylaxie ou la thérapeutique du cancer.

**6.** Utilisation selon la revendication 5, dans laquelle le médicament comprend un mélange d'IFN-$\beta$ et d'IL-2.

**7.** Utilisation selon la revendication 5, dans laquelle le médicament est une préparation combinée pour l'administration séparée d'IFN-$\beta$ et d'IL-2.

**8.** Produits contenant de l'IFN-$\beta$ et de l'IL-2 sous forme d'une préparation combinée pour l'administration simultanée, séparée ou successive dans un traitement thérapeutique ou prophylactique quelconque d'un cancer humain qui utilise l'IFN-$\beta$ et 1'IL-2 aux concentrations définies dans la revendication 1.

## Patentansprüche

**1.** Arzneizubereitung, geeignet zur Verabreichung an menschliche Patienten zur therapeutischen oder prophylaktischen Behandlung von Krebs, enthaltend IFN-beta und IL-2, jeweils in einem Trägerstoff und in Konzentrationen von etwa 0,1 mg/ml bis 100 mg/ml, wobei das IFN-beta und IL-2 aus Säugetier-Arten stammen.

**2.** Arzneizubereitung nach Anspruch 1, **dadurch gekennzeichnet,** daß das IFN-beta und IL-2 humanes rekombinantes IFN-beta und IL-2 sind.

**3.** Arzneizubereitung nach Anspruch 1 oder 2, **dadurch gekennzeichnet,** daß das IFN-beta IFN-beta$_{ser17}$ und das IL-2 das des-ala$_1$-IL-$_{ser125}$ ist.

**4.** Arzneimittelzubereitung nach einem der Ansprüche 1 bis 3, **dadurch gekennzeichnet,** daß der Krebs einen Darmkrebs, ein Melanom, Nierenzellenkrebs, Lungenkrebs, ein Adenokarzinom oder einen Brustkrebs darstellt.

**5.** Verwendung von Säugetier-IFN-beta und -IL-2 in den in Anspruch 1 angegebenen Konzentrationen zur Herstellung eines Arzneimittels zur Prophylaxe oder Behandlung von Krebs.

**6.** Verwendung nach Anspruch 5, **dadurch gekennzeichnet,** daß die Arzneizubereitung ein Gemisch aus dem IFN-beta und IL-2 enthält.

**7.** Verwendung nach Anspruch 5, **dadurch gekennzeichnet,** daß das Arzneimittel eine kombinierte Zusammensetzung zur getrennten Anwendung von LFN-beta und IL-2 ist.

**8.** IFN-beta und IL-2 enthaltende Produkte in Form einer kombinierten Zusammensetzung zur gleichzeitigen, getrennten oder nacheinander erfolgenden Verwendung bei jeglicher therapeutischer oder prophylaktischer Behandlung von menschlichem Krebs, wobei das genannte IFN-beta und Il-2 in den in Anspruch 1 definierten Konzentrationen eingesetzt werden.